# EUROPÄISCHE PATENTSCHRIFT

(11) **EP 0 933 090 B1**
(45) Veröffentlichungstag und Bekanntmachung des Hinweises auf die Patenterteilung: **19.07.2006**
(21) Anmeldenummer: 99101030.7
(22) Anmeldetag: 21.01.1999
(51) Int. Cl.: A61M 1/02, A61J 1/10

(54) **Blutbeutelsystem zur Pathogeninaktivierung von Blut, Blutkomponenten und Plasma**
Blood bag system for the inactivation of pathogens in blood, blood components and plasma
Système de poches de sang pour l'inactivation de pathogènes dans le sang, les composants sanguins et le plasma

(30) Priorität: 30.01.1998 DE 29801590 U
(43) Veröffentlichungstag der Anmeldung: 04.08.1999
(73) Patentinhaber: Maco Pharma International GmbH, 63225 Langen (DE)
(72) Erfinder: Coudaliez, Francis, c/o Maco Pharma S.A., 59338 Tourcoing (FR); Walker, Dr.Wolfram H., 63222 Rödermark (DE)
(74) Vertreter: Derambure, Christian

(56) Entgegenhaltungen:
- WO-A-87/06119
- WO-A-92/15274
- WO-A-97/18844
- WO-A-97/22245
- US-A- 4 971 991

## Beschreibung

Gegenstand der Erfindung ist ein Blutbeutelsystem gemäß den Merkmalen des Oberbegriffs des Anspruchs 1.

Menschliches Vollblut wird bei der Blutspende in dem primären Blutauffangbeutel eines Blutbeutelsystems gesammelt, um anschließend eine Aufteilung in die einzelnen Hauptkomponenten vornehmen zu können. Dieser primäre Blutbeutel enthält üblicherweise die primäre Blutstabilisatorlösung CPD. Das Blut in dem primären Beutel wird nach dem Stand der Technik durch Zentrifugation des gesamten Beutelsystems in die 3 Bestandteile Plasma (oben), buffy-coat (Mitte), das u.a. die Thrombozyten und teilweise Leukozyten enthält, und in Erythrozytenkonzentrat (unten) aufgetrennt. Diese 3 Komponenten werden nach der Zentrifugation üblicherweise in die mit dem primären Blutbeutel steril verbundenen Beutel des Blutbeutelsystems gepreßt.

Es sind Beutelsysteme beschrieben, bei denen das Erythrozytenkonzentrat, bevor es in den für seine Aufnahme vorgesehenen Beutel fließt, durch ein Leukozytenfilter filtriert wird. Hierbei befindet sich das Filter in der Schlauchleitung zwischen den Präparationsbeuteln. Eine Broschüre der Firma Maco Pharma International GmbH, präsentiert im September 1995, beschreibt verschiedene Blutbeutelsysteme, bei denen sich ein Leukozytenfilter im geschlossenen Blutbeutelsystem befindet.

Im deutschen Gebrauchsmuster G 296 03 873.3 werden Blutbeutelsysteme beschrieben, in denen der Leukozytenfilter zwischen dem primären Beutel, der das Vollblut aufnimmt und den Beuteln, die zur Aufnahme des Plasmas und des Erythrozytenkonzentrates bestimmt sind, angeordnet ist.

In PCT/DE92/00031 (WO 92/15274) wird eine Blutbeutelanordnung beschrieben, die in abgeschlossenen Teilräumen des Beutelsystems eine oder mehrere Substanzen enthält, die zur photoaktiven Virusinaktivierung von Blutkomponenten dienen und durch die das Blut bzw. die Blutbestandteile einzelner Spender virussicher gemacht werden können.

WO-97/18844 offenbart ein Blutbeutelsystem zur Auftrennung von Vollblut, bestehend aus einem Leukozytenfilter dem gegebenenfalls ein Auffangbeutel vorgeschaltet ist und einem damit über sterile Schläuche verbundenen Inaktivierungsbeutel, wobei stromabwärts des Leukozytenfilters ein Behälter angeordnet ist, der mindestens eine Substanz zur Pathogeninaktivierung von Blut oder Blutkomponenten enthält.

Ziel der Erfindung ist es, ein Blutbeutelsystem zur Auftrennung des Vollblutes in seine Komponenten zur Verfügung zu stellen, das gleichzeitig die Leukozytenfiltration und die Inaktivierung von Viren, Keimen bzw. Pathogenen durch eine oder mehrere Substanzen, integriert in einem Beutelsystem, erlaubt. Ziel ist es außerdem, ein praktikables, steriles Beutelsystem zur Verfügung zu stellen, bei dem eine Leukozytenfiltration des Vollblutes oder der einzelnen Blutkomponenten erfolgt und bei der die im Beutel befindlichen Substanzen zur Pathogeninaktivierung in Pillenform vorliegen. Dies hätte u.a. den Vorteil, daß eine bessere Lagerstabilität dieser Substanzen möglich ist, wodurch eine lange Lagerbarkeit des Beutelsystems vor der Anwendung ermöglicht wird. Ziel der Efindung ist es ferner, gegebenenfalls die zur Inaktivierung verwendeten Substanzen noch in dem geschlossenen Beutelsystem wieder zu entfemen.

Diese Ziele werden durch die in den kennzeichnenden Teile der Ansprüche definierten Anordnungen erreicht. Anhand der beigefügten Zeichnungen wird die Erfindung näher beschrieben.

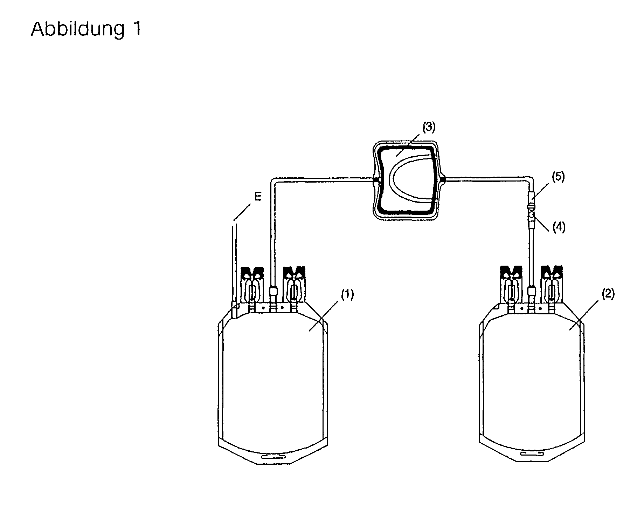

Abb. 1 zeigt ein Beutelsystem, bei dem der Filter (3) vor dem Inaktivierungsbeutel (2) angeordnet ist, wobei in diesem Fall dem System ein (nicht zwingend notwendiger) Auffangbeutel (1) vorgeschaltet ist, und der Behälter (4) mit der Substanz zur Pathogeninaktivierung in Form einer Pille sich in einem sog. Abbrechventil (5) des Schlauches zwischen Filter (3) und Beutel (2) befindet. Erst nach Öffnen des Abbrechventils läuft das dem Auffangbeutel (1) Ober die Zuleitung (E) zugeführte Blut oder die Blutkomponente vom Auffangbeutel (1) über den Filter (3) in den Inaktivierungsbeutel (2). Die Substanzen werden während der Passage aufgelöst und in den Beutel (2) gespült. Das in dieser Abb. 1 gezeigte Beutelsystem dient sowohl zur Filtration und Inaktivierung von Vollblut als auch von Blutkomponenten. Im Falle der Inaktivierung von Vollblut wird das Blut nach Füllung Ober den Entnahmeschlauch (E) direkt über ein Vollblut-Leukozytenfilter in den Beutel (2) geleitet, nachdem das Abbrechventil (5) geöffnet wurde. Im Falle des Humanplasmas, wird das Doppelbeutelsystem zunächst über den Schlauch (E) an einen Beutel oder ein Beutelsystem gekoppelt, indem sich das Plasma befindet und anschließend, wie für Vollblut beschrieben, zusammen mit Substanz aus dem Behälter (4) in den Inaktivierungsbeutel (2) gespült. Vorzugsweise wird bei Humanplasma als Filter (3) ein Membranfilter eingesetzt. Es kann jedoch auch ein Mehrschichtenfilter mit Membranfilter oder eine Kombination von Tiefen- u. Membranfliter eingesetzt werden. Dieses Membranfilter kann sowohl In einem starren als auch flexiblen Gehäuse plaziert sein. Ähnliche flexible Filter sind in der oben erwähnten Broschüre der Firma Maco Pharma erwähnt.

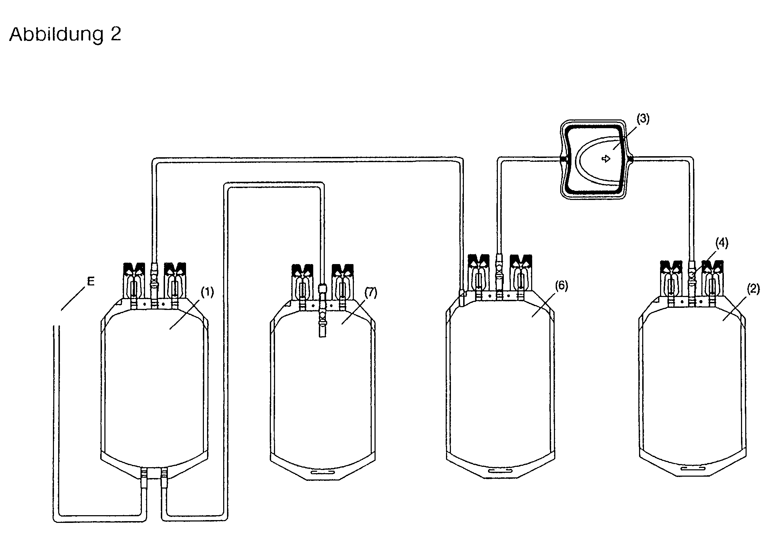

Abb. 2 zeigt ein Mehrbeutelsystem, bestehend einem über die Zuleitung (E) mit Spenderblut befüllbarem Auffangbeutel (1) dem Filter (3) und dem Inaktivierungsbeutel (2). Am Auffangbeutel (1) befindet sich ein Abgang nach unten zu dem Beutel (7), der im allgemeinen eine Erythrozytenkonservierungslösung enthält sowie einen Abgang nach oben zu dem Zwischenbeutel (6), in den das Plasma abgepreßt wird. Die Substanzen zur Inaktivierung können sich sowohl im Schlauch zwischen Filter (3) und dem Sammelbeutel (2) als auch am Eingang dieses Beutels (2) oder direkt im Beutel (2) oder auch im Filter (3) befinden.

Nach Zentrifugation des im Beutel (1) befindlichen Blutes wird das Plasma in den Beutel (6) gepreßt und anschließend über den Leukozyten-Filter (3) in den Beutel (2) geleitet. Die Pathogeninaktivierungs-Substanz wird hierbei mit dem Plasma aufgelöst bzw. vermischt und zusammen mit dem Plasma in den Beutel (2) transferiert. Somit erhält man ein Plasma, das einerseits filtriert und gleichzeitig mit Hilfe dieser Substanzen virus-/pathogeninaktiviert wird.

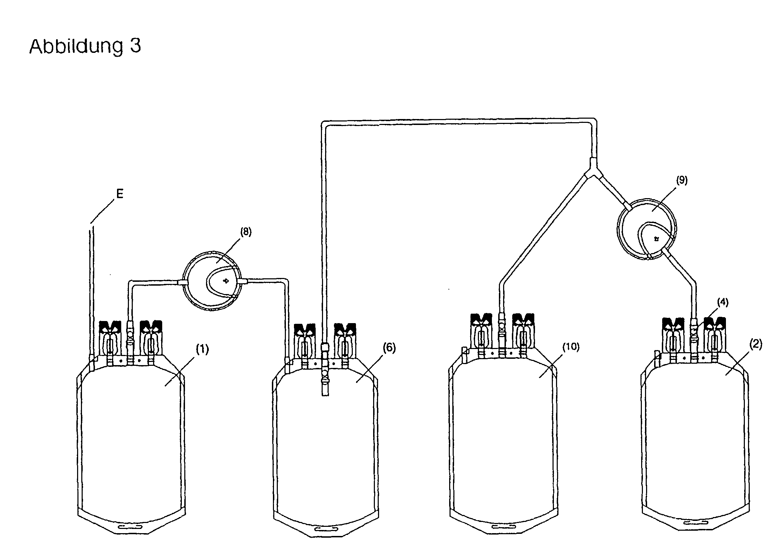

Abb. 3 zeigt ein Beutelsystem mit einer ähnlichen Anordnung. Hier ist jedoch zwischen dem Auffangbeutel (1) und dem Zwischenbeutel (6) bereits ein erster Leukozytenfilter (8) angeordnet. Da das gesamte Blut schon einer Leukozytenfiltration mit Hilfe des Filters (8) unterzogen worden ist, kann der Filter (9) ein Spezialfilter, z.B. ein 0,25 µm Sterilfilter oder 0,5 - 1,0 µm Membran-Filter zur Filtration des Plasmas sein. Dieser Filter kann, bedingt durch die primäre Leukozytenfiltration des Blutes durch Filter (8), auch wesentlich kleiner hinsichtlich der Größe und wirksamen Oberfläche sein.

Wie bereits bei der Erläuterung der Abb. 1 erwähnt, kann in Abhängigkeit von den Bedingungen des Einzelfalls der Auffangbeutel (1) entfallen und das Blut bzw. die Blutkomponente dem restlichen System direkt zugeführt werden.

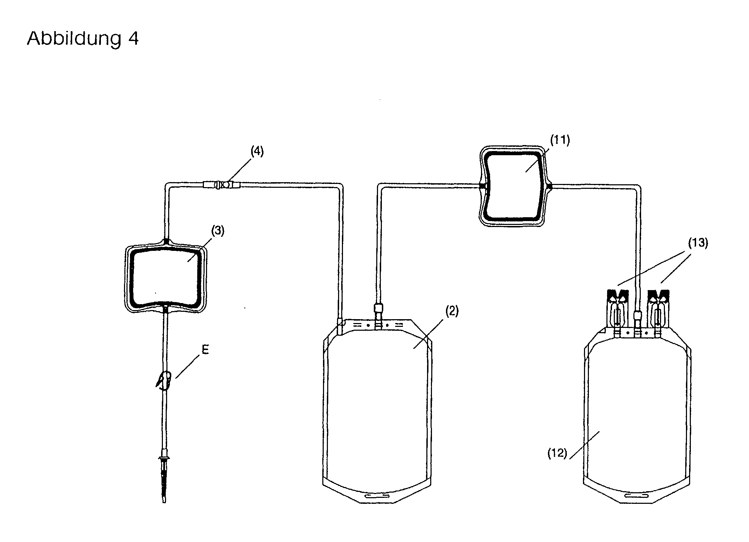

Abb. 4 zeigt ein weiteres bevorzugtes Beutelsystem ähnlich dem System der Abb. 1, jedoch ohne einen Auffangbeutel (1), mit dem Plasmafilter (3), der Pathogeninaktivierungs-substanz (4) und den Inaktivierungsbeutel (2). Dieser Beutel (2) ist über eine zusätzliche Trennvorichtung (11) mit einem Transfusionsbeutel (12) verbunden. Diese Trennvorrichtung (11) dient zum Entfernen der Inaktivierungssubstanz aus der Blutkomponente oder dem Plasma. Dies erlaubt eine Transfusion des in diesen Transfusionsbeutel (12) gelangenden Blutprodukts direkt aus diesem Beutel. Die Trennvorrichtung (11) kann aus einem geeigneten Adsorptionsmaterial oder einem Spezialfilter bestehen.

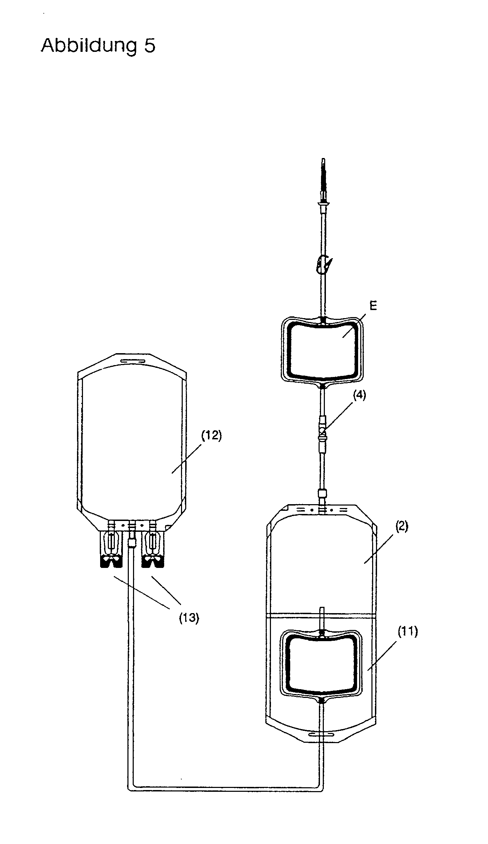

Abb. 5 zeigt eine Variante der Abb. 2 oder 4, bei der sich das Adsorptions- bzw. Filtermaterial (11) im unteren Teil des Inaktivierungsbeutels (2) befindet. Bei der photodynamischen Virusinaktivierung von Plasma durch Methylenblau wird das mit Methylenblau versetzte Plasma, das sich im Beutel (2) befindet, durch Bestrahlen inaktiviert. Nach der Inaktivierung wird das Plasma über den Filter (11) in den Beutel (12) geleitet. Der Filter (11) ist in diesem Fall ein spezieller Methylenblau-Adsorptionsfilter. Im Beutel (12) steht deshalb ein von Methylenblau befreites Plasma zur Transfusion bereit. Der Filter (11) kann sich direkt im unteren Teil des Inaktivierungsbeutels (2) oder in einer zweiten Kammer dieses Beutels (2) befinden, wie diese Abb. 5 andeutet.

Die in den Abb. 4 und 5 gezeigten Gestaltungen des Beutelsystems können über die Zuleitung (E) mit anderen Komponentenbeuteln ähnlich den in Abb. 2 und 3 dargestellten Lösungsformen verbunden sein oder steril an diese angedockt werden.

Vorzugsweise enthält der letzte Beutel des erfindungsgemäßen Beutelsystems die zur Infusion notwendigen Stutzen (13) sowie Möglichkeiten der Kennzeichnung, beispielsweise durch entsprechende Etikettierung.

Das erfindungsgemäße Beutelsystem, bestehend aus einer Kombination von Blut- bzw. Blutkomponentenfilter, Inaktivierungssubstanz und Inaktivierungsbeutel hat den Vorteil, daß sämtliche Präparationsschritte, wie Blutkomponentenpraparation, Leukozytenfiltration, Dosierung und Durchführung der Inaktivierung, im geschlossenen System durchgeführt werden. Im Falle der photodynamischen Virusinaktivierung von Plasma mit Methylenblau wird das gesamte System einer Dampfsterilisation unterzogen. Die erwähnte Pille, enthält neben Methylenblau, Polyol, Glucose und Zitrat. auch Polyäthylenglycol oder Mannitol und ggf. andere Zusatzstoffe, die eine Stabilität während der Sterilisation erlauben, aber gleichzeitig eine gute Herstellung, Dosierung und Lösbarkeit der Pille gewährleisten. Der Vorteil des in Abb. 1 dargestellten bevorzugten Systems ist dessen günstige Herstellbarkeit sowie die leichte und günstige Handhabung von Filtration, Zudosierung virusaktiver Substanzen sowie gegebenenfalls der anschließenden photodynamischen Bestrahlung des gefüllten Präparatebeutels sowie gegebenenfalls des Entfernens der Inaktivierungssubstanz. Ein weiterer Vorteil des Systems besteht bei der Verwendung eines flexiblen Membranfilters als Filter darin, daß die bei der Präparation im Inaktivienrngsbeutel (2) gesammelte sterile Luft Ober das Schlauchsystem in den flexiblen Filter zurückgepreßt werden kann. Dies ist bei starren. Filtern nicht möglich, dort ist deshalb ein Bypass oder eine separate Entlüftung, die geöffnet und geschlossen werden muß, erforderlich. Eine Entfernung der im Beutel (2) vorhandenen Luft ist sowohl für die Durchführung einer photodynamischen Virusinaktivierung als auch bei der Transfusion des fertigen Produkt erforderlich.

Die virusinaktivierende Substanz kann beispielsweise eine photodynamisch wirksame Agens, wie Methylenblau, enthalten. In diesem Falle wird anschließend das im Inaktivierungsbeutel (2) befindliche Plasma einer photodynamischen Bestrahlung unterzogen. Bei der photodynamischen Bestrahlung wird der mit Plasma und virusinaktivierenden Substanzen gefüllte Inaktivierungsbeutel (2) belichtet. Durch diese Belichtung oder Bestrahlung findet mit Hilfe der photodynamischen Substanzen eine Virusinaktivierung des Plasmas statt. Die virusinaktivierende Substanz besteht in diesem Fall vorzugsweise aus einer Pille, die neben Methylenblau u.a. Glucose, Mannitol oder Zitrat enthält. Eine günstige Dosierung und ein gutes Auflösungsverhalten dieser Substanz ist beispielsweise in Form einer Pille, bestehend aus Methylenblau und Mannitol, möglich. Dadurch löst sich die Pille bereits während des Plasmaflusses von Filter (1) in Beutel (2) auf. Auch die Plazierung der Pille mit der Wirksubstanz in den Bereich des Abbrechventils (5) hat Vorteile, weil das Abbrechteil durch z.B. Verklebung oder Verschweißung eingebracht werden kann. Eine selektive Bestückung des Raumes im Bereich des Abbrechventils mit Pillenforms begünstigt eine Handhabung dieser Substanzen. So kann z. B. das Abbrechventil (5) zusammen mit der Wirksubstanz vorab einer Sterilisation, z.B. β- oder γ-Sterilisation, unterzogen werden, bevor das Teil in das Beutelsystem eingebracht wird. Anschließend werden die komplettierten erfindungsgemäßen Blutbeutelsysteme einer Dampfsterilisation unterzogen, da die Präparation, Filtration, Inaktivierung sowie Entfernung der Inaktivierungssubstanz unter sterilen Bedingungen erfolgen muß.

## Patentansprüche

1. Blutbeutelsystem zur Auftrennung von Vollblut, beinhaltend eine Laukozytenfilter (3), dem gegebenenfalls ein Auffangbeutel (1) vorgeschaltet ist, einem damit über sterile Schläuche verbundenen inaktivierungsbeutel (2), und wobei das Blutbeutelsystem mindestens eine Substanz zur Pathogeninaktivierung von Blut oder Blutkomponenten in Flussrichtung des Blutes- oder der Blutkomponenten hinter dem Leukozytenfilter (3) enthält, **dadurch gekennzeichnet, dass** die Substanz in Pillenform vorliegt und zusätzlich mindestens Mannitol oder Polyäthylenglycol enthält.

2. Beutelsystem nach Anspruch 1, **dadurch gekennzeichnet, daß** sich die Substanz zur Inaktivierung im Inaktivierungsbeutel (2) befindet.

3. Beutelsystem nach Anspruch 1, **dadurch gekennzeichnet, daß** die Substanz in einem Behälter (4) sich befindet, der an Eingang des Inaktivierungsbeutels (2) oder im Zuleitungsschlauch zu diesem Beutel angeordnet ist.

4. Beutelsystem nach Anspruch 1, **dadurch gekennzeichnet, daß** sich der Behälter (4) im einem Abbrechventil (5) des Beutelsystems angeordnet ist.

5. Beutelsystem nach einem der Ansprüche 1 bis 4, **dadurch gekennzeichnet, daß** der Filter (3) ein Tiefenfilter oder Membranfilter oder eine Kombination beider ist.

6. Beutelsystem nach Anspruch 5, **dadurch gekennzeichnet, daß** der Filter ein Membran-filter mit zwischen 0,2 - 1,0 µm ist

7. Beutelsystem nach einem der Ansprüche 1 bis 6, **dadurch gekennzeichnet, daß** die Substanz zusätzlich Glucose oder Zitrat zusammen mit einer Lösungshilfe enthält

8. Beutelsystem nach Anspruch 7, **dadurch gekennzeichnet, daß** die Pille Methylenblau und Mannitol sowie Lösungsvermittlern enthält.

9. Beutelsystem nach einem der Ansprüche 1 bis 8, **dadurch gekennzeichnet, daß** zusätzlich eine Trennvorrichtung (11) zum Entfernen der Inaktivierungssubstanz sowie dieser nachgeschaltet ein Transfusionsbeutel (12) vorhanden sind.

10. Beutelsystem nach Anspruch 9, **dadurch gekennzeichnet, daß** die Trennvorrichtung (11) in der Schlauchleitung zwischen dem Inaktivierungsbeutel (2) und dem Transfusionsbeutel (12) angeordnet ist.

11. Beutelsystem nach Anspruch 9, **dadurch gekennzeichnet, daß** die Trennvorrichtung (11) im unteren Teil des Inaktivierungsbeutels (2) angeordnet ist.

12. Beutelsystem nach Anspruch 10, **dadurch gekennzeichnet, daß** die Trennvorrichtung (11) in einer unteren zweiten Kammer des Inaktivierungsbeutels (2) angeordnet ist.

## Claims

1. Blood bag system for the separation of whole blood, comprising a leukocyte filter (3), a collecting bag (1) which may be disposed there before, an inactivation bag (2) connected to the filter through a sterile tubing and in which the blood bag system contains at least one substance for inactivation of pathogens from blood or blood components placed behind the leukocyte filter (3) in the flow direction of the blood or blood components, **characterised in that** the substance shows a pill shape and contains at least mannitol and polyethylene glycol.

2. Bag system according to claim 1, **characterised in that** the substance for inactivation of pathogens is located in the inactivation bag (2).

3. Bag system according to claim 1, **characterised in that** the substance is located in a container (4) arranged in the inlet of the inactivation bag (2) or in the tubing leading to this bag.

4. Bag system according to claim 1, **characterised in that** the container (4) is arranged in a break-off valve (5) of the bag system.

5. Bag system according to claims 1 to 4, **characterised in that** the filter is a depth filter or a membrane filter or a combination thereof.

6. Bag system according to claim 5, **characterised in that** the filter (3) is a membrane filter between 0.2 and 1.0 µm.

7. Bag system according to claims 1 to 6, **characterised in that** the substance contains glucose or citrate with a dissolution aid.

8. Bag system according to claim 7, **characterised in that** the pill contains methylene blue and mannitol as well as dissolution agents.

9. Bag system according to claims 1 to 8, **characterised in that** a separation device (11) for the removal of the inactivation substance and a hereafter disposed transfusion bag (12) are also present.

10. Bag system according to claim 9, **characterised in that** the separation device (11) is arranged in the tubing conduct between the inactivation bag (2) and the transfusion bag (12).

11. Bag system according to claim 9, **characterised in that** the separation device (11) is arranged inside a bottom part of the inactivation bag (2).

12. Bag system according to claim 10, **characterised in that** the separation device (11) is arranged inside a second bottom chamber of the inactivation bag (2).

## Revendications

1. Système de poches à sang pour la séparation du sang total, comportant un filtre à leucocytes (3), éventuellement une poche de recueil (1) placée avant celui-ci, une poche d'inactivation (2) reliée au filtre au moyen de tubulures stériles et dans lequel le système de poches à sang contient au moins une substance pour l'inactivation des pathogènes du sang ou des composés sanguins placée après le filtre à leucocytes (3) dans le sens d'écoulement du sang ou du composé sanguin, **caractérisé en ce que** la substance se présente sous forme de pilule et qu'elle contient au moins du mannitol ou du polyéthylène glycol.

2. Système à poches selon la revendication 1, **caractérisé en ce que** la substance pour l'inactivation se trouve dans la poche d'inactivation (2).

3. Système à poches selon la revendication 1, **caractérisé en ce que** la substance est agencée dans un conteneur (4) disposé à l'entrée de la poche d'inactivation (2) ou dans la tubulure d'amenée de cette poche.

4. Système à poches selon la revendication 1, **caractérisé en ce que** le conteneur (4) est agencé dans un ouvre-circuit (5) du système à poches.

5. Système à poches selon les revendications 1 à 4, **caractérisé en ce que** le filtre (3) est un filtre en profondeur ou un filtre membrane ou une combinaison des deux.

6. Système à poches selon la revendication 5, **caractérisé en ce que** le filtre est un filtre membrane entre 0,2 et 1,0 µm.

7. Système à poches selon les revendications 1 à 6, **caractérisé en ce que** la substance contient en plus du glucose ou du citrate additionné d'un auxiliaire de solubilisation.

8. Système à poches selon la revendication 7, **caractérisé en ce que** la pilule contient du bleu de méthylène et du mannitol ainsi que des agents de solubilisation.

9. Système à poches selon les revendications 1 à 8, **caractérisé en ce que** sont également présents un dispositif de séparation (11) pour l'élimination de la substance d'inactivation ainsi qu'une poche de transfusion (12) placée après celui-ci.

10. Système à poches selon la revendication 9, **caractérisé en ce que** le dispositif de séparation (11) est agencé dans la tubulure entre la poche d'inactivation (2) et la poche de transfusion (12).

11. Système à poches selon la revendication 9, **caractérisé en ce que** le dispositif de séparation (11) est agencé dans une partie basse de la poche d'inactivation (2).

12. Système à poches selon la revendication 10, **caractérisé en ce que** le dispositif de séparation (11) est agencé dans un deuxième compartiment bas de la poche d'inactivation (2).
